# EUROPEAN PATENT APPLICATION

(11) **EP 4 295 830 A1**
(43) Date of publication of application: **27.12.2023**
(21) Application number: 22179947.1
(22) Date of filing: 20.06.2022
(51) Int. Cl.: A61F 15/00, A61B 50/37

(54) **A PACKAGE COMPRISING MEDICAL DRESSINGS**

(71) Applicant: Mölnlycke Health Care AB, 402 52 Göteborg (SE)
(72) Inventor: HAMMAR, Elin, KULLAVIK (SE); MÅRTENSSON, Malin, BORÅS (SE); LINDBERG, Rasmus, GÖTEBORG (SE); STRÖM, Caroline, GÖTEBORG (SE); URSTEN RIEMAN, Pia, GÖTEBORG (SE)
(74) Representative: Kransell & Wennborg KB

(57) **Abstract**

The present disclosure generally relates to a package (100) comprising a plurality of medical dressings, wherein the package (100) comprises a front panel (101), an opposing back panel (102) and four side panels (103a-d) extending between the front panel (101) and the back panel (102), wherein the package comprises a set of printed product identifying elements (104a-e, 105a-e, 106a-e, 107a-e) associated with the medical dressings in the package, wherein the set of printed product identifying elements comprises at least a first text element (104a-e), preferably a brand name, and a first graphical element (105a-e) visualizing a medical dressing in the package (100), wherein the first text element (104a-e) and the first graphical element (104a-e) are disposed on the front panel (101) and on each one of the side panels (103a-d) of the package (100).

## Description

### TECHNICAL FIELD

The present disclosure generally relates to a package comprising a plurality of medical dressings, wherein the package comprises a front panel, an opposing back panel and four side panels extending between the front panel and the back panel, wherein the package comprises a set of printed product identifying elements associated with the medical dressings in the package. The present disclosure also relates to a flat box for packaging medical dressings.

### BACKGROUND

Medical dressings come in a huge variety of shapes, sizes and designs. Furthermore, medical dressings may be used for an endless number of purposes and in a variety of different wound related contexts. For example, medical dressings may be used for burns, scars, incisions, open wounds, chronic wounds, acute wounds, and also for preventative purposes; i.e. for the purpose of preventing a wound from occurring in the first place.

Medical dressings may be associated with various characteristics, such as the degree of absorption, adhesion, antimicrobial (or non-antimicrobial) etc., and are available in a vast variety of sizes. Even within a specific category of medical dressings, a huge number of different characteristics is generally available.

One example of a category of dressings is Mepilex^{®} foam dressings. Mepilex^{®} foam dressings come in a plurality of sub-brands, e.g. Mepilex^{®} Ag, Mepilex^{®} XT, Mepilex^{®} Border, Mepilex^{®} Lite etc. Within each one of the sub-branded dressing categories, a large variation of different dressing sizes and dressing characteristics is available.

The endless number of uses and characteristics associated with medical dressings within a specific dressing category may cause confusion, and also a burden for the applicators of the dressings, typically caregivers or medical personnel, with respect to what dressing to use for a specific wound in a specific care scenario.

Prior to use, medical dressings are typically individually packaged in sterile pouches, which in turn are packaged in containers housing a plurality of such sterile pouches.

In a hospital or care facility, the containers, or packages, comprising the medical dressings, are often stored on shelves in a separate storage room. Usually, a vast number of packages are stored together. Since many wound dressing packages have a visually similar appearance, the packages, and the particular medical dressings stored therein, are difficult to distinguish from one another. Accordingly, in various care scenarios, which are often associated with a sense of urgency, the selection of the correct dressing may become a time-consuming and complicated task.

There is consequently a need to facilitate the burden for caregivers and medical personnel, and to provide a means to easily identify and select the correct, or sometimes just the most promising, medical dressing to be used for a specific patient in a specific care scenario.

### SUMMARY

In view of the above-mentioned problems, it is an object of the present disclosure to provide improvements with respect to relieving the stress for caregivers in various daily care situations and providing a facilitated, clear and communicative means to guide the staff and caregivers to a correct product selection and use.

According to a first aspect, there is provided a package comprising a plurality of medical dressings, wherein the package comprises a front panel, an opposing back panel and four side panels extending between the front panel and the back panel, wherein the package comprises a set of printed product identifying elements associated with the medical dressings in the package, wherein the set of printed product identifying elements comprises at least a first text element, preferably a brand name, and a first graphical element visualizing a medical dressing in the package, wherein the first text element and the first graphical element are disposed on the front panel and on each one of the side panels of the package.

In hospitals and care facilities, packages containing medical dressings are typically stored and arranged on shelves such that one of the panels is facing the room and the viewer. By visualizing the medical dressing contained in the package on each one of the side panels of the package, the specific product; i.e. dressing contained in the package is visualized and easily recognizable regardless of how the package is stored in the shelf, and regardless of which side panel faces the caregiver or medical personnel.

The text element is typically the brand name; i.e. the main product brand. By visualizing the medical dressing in conjunction with the brand name, the recognition of the medical dressing contained in the package is improved.

In cases where the medical personnel takes out one or more dressings from the package and thereafter places the package back in the shelf such that a different side panel is facing the room/viewer, such mis-placement is rectified by the presence of the first graphical element; i.e. the medical dressing and the text element on each one of the side panels.

It is further believed that the present disclosure provides improvement on how products can be organized with respect to product ranges and product types, thereby facilitating a clearer and more communicative means to guide the staff and caregivers to a correct product selection and use.

The first graphical element may be an image, preferably a photograph, of the medical dressing.

Preferably, the first graphical element is a colored photograph or image of the dressing, which improves the recognition of the product during storage.

In exemplary embodiments, the set of printed product identifying elements comprises at least a second graphical element indicating a dressing property, preferably the absorption level, associated with the medical dressings comprised in the package, and wherein the second graphical element is disposed on the front panel and on each one of the side panels of the package.

The second graphical element can be representative of one product property encompassed by the medical dressing in the package, or at least one product property encompassed by the medical dressing in the package.

The product property is preferably the absorption level associated with the medical dressings in the package.

The absorption level may be visualized in a number of ways, but is preferably indicated with a pictorial representation. The pictorial representation may convey information about the absorption level in a manner that allows the caregiver and personnel to select a "low absorption", "medium absorption" or "high absorption" dressing.

In exemplary embodiments, the set of printed product identifying elements comprises at least a second text element indicating the size of the medical dressings in the package, and wherein the second text element is disposed on the front panel and on each one of the side panels of the package.

Accordingly, the size of the dressing is displayed in a clear manner and is visible to the caregivers and/or personnel regardless of how the package is positioned on a storage shelf. Accordingly, the size of the product is clearly identifiable to the personnel. Quick decisions may therefore be made as to what medical dressing is suitable for use in a specific care situation.

In exemplary embodiments, the front panel and/or the side panel(s) comprises at least one color coded portion; the color coded portion being indicative of a specific medical dressing category.

The color coded portion improves and facilitates the selection of a specific dressing category. The color coded portion may indicate a particular genre or category of medical dressings, e.g. antimicrobial dressings, light or high absorbing dressings, etc.

In exemplary embodiments, the color coded portion is correlated with the first graphical element or the second graphical element.

For example, in embodiments where the package comprises antimicrobial medical dressings, the color coded portion may be grey. The grey color may, by medical personnel, be associated with antimicrobial properties. Typically, antimicrobial wound dressings comprise silver as the antimicrobial agents, and the color of silver containing wound pads is typically grey. In embodiments where the first graphical element is visualized as a color image or colored photograph, the wound pad of the medical dressing illustrated on the front panel of the package is grey. Accordingly, the combined grey colored dressing and grey color coded portion yields an enhanced impression and identification of the specific dressing category. It is consequently clear to the personnel which packages contain antimicrobial dressings, such that this dressing category can be distinguished from other dressing categories.

Alternatively, the color coded portion may be associated with the second graphical element, preferably indicating the absorption level of the dressing. A higher absorption level may be associated with a sharper color. A lower absorption level may be associated with a lighter color. Typically, the same color is utilized for high absorbing and low absorbing medical dressing, but the shade of the color varies.

For example, the color coded portion may be light green if the absorption level is low, and green or dark green if the absorption level is high.

Preferably, each one of the side panels comprises a color coded portion, the color coded portion being indicative of a specific medical dressing category.

By disposing the color coded portion on each one of the side panels of the package, the medical personnel are offered a clear and easily recognizable product identification, which significantly facilitates the selection of a specific type of dressing.

To further improve the product selection and visual impression of the package, the first graphical element disposed on each one of the side panels is arranged in the color coded portion.

In exemplary embodiments, the side panels have a background color that contrasts with the background color of the front panel, and, optionally, of the back panel.

The front panel typically has a lighter background color, e.g. white. This is beneficial since it allows for a plurality of product identifying elements; i.e. text elements and graphical elements on the front panel to be visualized and recognized.

The side panels preferably have a color that contrasts with the lighter background color of the front panel. For example, the background color may be green, purple, pink, blue, green, red and any shades thereof.

The color contrast between the front panel and the side panels yields a visually appealing and more cohesive impression. It allows the caregivers and medical personnel to easily recognize the products connected to a primary brand and/or a specific supplier, when the packages are stored together with various other dressing or wound care product packages.

It may further assist in sorting the products and organize them in a way that permits easy recognition of the products.

For example, the background color of the side panels may be green and the background color of the front panel, and, optionally, the back panel may be white.

The green color of the side panels allows for a product and/or company recognition, and may distinguish the packages from different packages from other suppliers and other brands.

In exemplary embodiments, the side panels are visually correlated.

Accordingly, each side panel yields a similar visual impression and conveys substantially the same product identifying elements and background color.

The visual correlation between the side panels secures that regardless of how the packages are stored or placed onto a shelf, the side panels, when stored in an array of packages, will yield a similar impression to a viewer.

In exemplary embodiments, the front panel comprises a larger number of printed product identifying elements than the side panels.

The front panel typically represents the surface that faces the viewer (i.e. caregiver) when a medical dressing is to be taken out of the package.

Preferably, a more comprehensive set of product identifying elements; i.e. a more comprehensive set of text elements and graphical elements are present on the front panel.

In exemplary embodiments, each one of the side panels contains four printed product identifying elements associated with the medical dressings, and wherein the product identifying elements are disposed on the side panels in a visually correlated arrangement.

It may be beneficial to keep the side panels as "clean" as possible, to avoid creating a confusing impression with "too much" information and too many text elements and graphical elements. The side panels typically contain the key product identifying elements, which presents the medical personnel with the most important information connected to the medical dressings in the package.

In exemplary embodiments, the set of product identifying elements comprises a tag being readable using an electronic device, wherein the tag is disposed on the front panel of the package.

Such a tag may also be referred to as a machine readable code.

The tag may be a bar code, or QR code, that links the applicator to a website where additional information about the use and application of the specific medical dressing in the package. Also, the medical personnel may gain access to additional information relating to which wounds are suitably treated with the specific medical dressing in the package.

In exemplary embodiments, the first graphical element disposed on the front panel visualizes at least a portion of the top layer and at least a portion of a bottom layer of the medical dressing.

Accordingly, the correct positioning of the product to a patient is easily understandable for the caregivers and medical personnel.

Each one of the medical dressings comprised in the package is typically individually stored in a sterile pouch.

The sterile pouch keeps the dressing in a sterile condition prior to use.

In exemplary embodiments, the sterile pouch comprises a second set of printed product identifying elements associated with the medical dressing, and wherein at least two product identifying elements of the second set of product identifying elements correspond to at least two product identifying elements of the set of printed product identifying elements of the package.

The second set of product identifying elements may also contain additional product descriptive information. For example, the second set of product identifying elements may comprise at least one pictorial representation of a step associated with the correct application of the medical dressing onto the body part.

The medical staff is thus offered a direct, "on site" and "in situation" assistance such that the dressing can be properly applied onto the body part of the patient.

According to a second aspect, a flat box for packaging of medical dressings is provided; the flat box comprising a front panel, a back panel, and four side panels, wherein the four side panels are arranged around the front panel or the back panel; the front panel being connected to the back panel by one of the four side panels, wherein the flat box comprises a set of printed product identifying elements associated with the medical dressings to be packaged in the flat box, wherein the set of printed product identifying elements comprises at least a first text element, preferably a brand name, and a first graphical element visualizing a medical dressing to be packaged in the flat box, wherein the first text element and the first graphical element are disposed on the front panel and on each one of the side panels of the flat box.

The provision of printed product identifying elements on the flat box facilitates sorting and assembly of the final packages comprising a specific type of dressings corresponding to the product identifying elements.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1 illustrates a perspective view of a package according to an exemplary embodiment of the present disclosure.
Figure 2 illustrates the package of figure 1 in the form of a flat box, and visualizes the front panel, back panel and each one of the side panels of the package.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

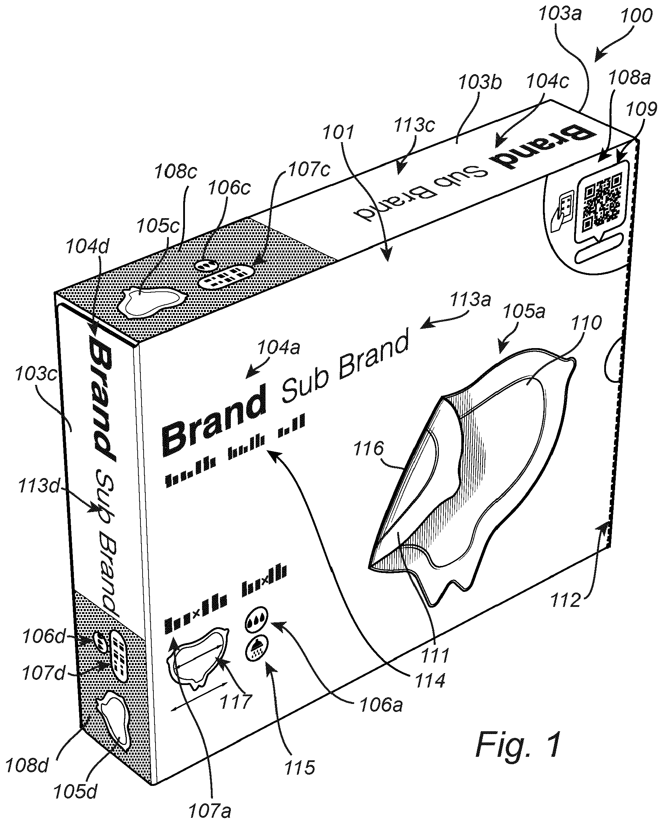

With reference to figure 1, a package 100 comprising a plurality of medical dressings, according to the present disclosure is conceptually illustrated.

The package 100 comprises a front panel 101, an opposing back panel and four side panels extending between the front panel 101 and the back panel 102, wherein the package comprises a set of printed product identifying elements associated with the medical dressings in the package, wherein the set of printed product identifying elements comprises at least a first text element, preferably a brand name, and at least a first graphical element visualizing a medical dressing in the package 100, wherein the first text element and the first graphical element are disposed on the front panel 101 and on each one of the side panels of the package.

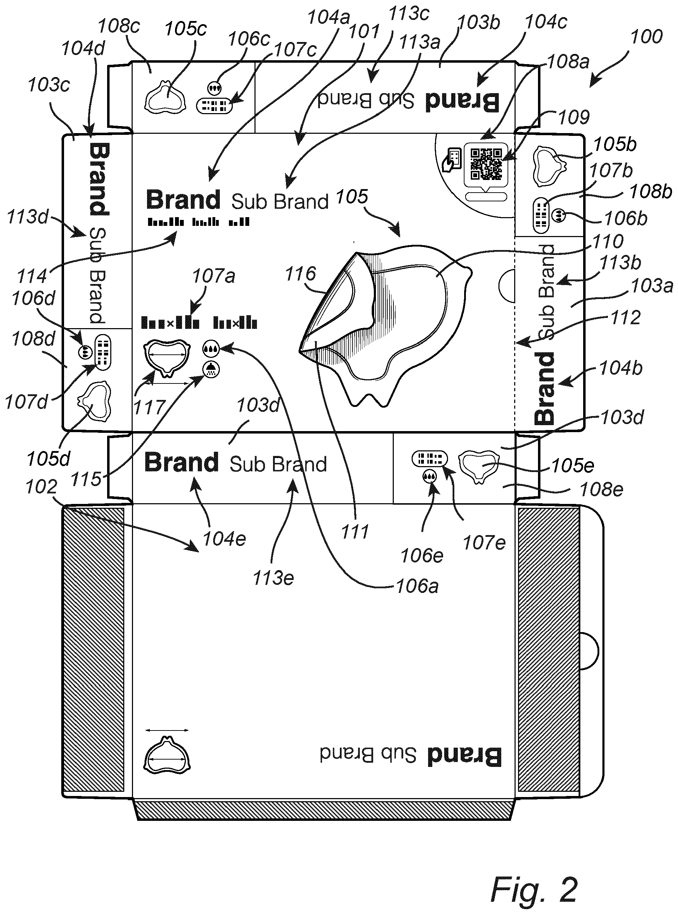

Figure 2 illustrates the package in a flat condition; i.e. in the form of a flat box, such that the front panel, back panel and side panels are shown.

The side panels are denoted 103a-d in figure 2, and the back panel is denoted 102.

The first text element 104a-e and the first graphical element 105a-e are disposed on each one of the side panels 103a-d, as best shown in figure 2.

The package is typically a carton, and may be formed from paper, e.g. cardboard. The package is typically square or rectangular in shape.

Each package typically comprises at least five medical dressings.

The "front panel" of the package may also be referred to as a top panel of the package. This is the panel that is viewable to the medical personnel when the package has been taken off the shelf and a medical dressing is to be taken out of the package.

The "back panel" of the package may be referred to as the bottom panel of the package.

The "side panels" of the package extend between the front panel and the back panel of the package. The side panels are typically the panels that are viewable to caregivers or medical personnel when the packages are stored on a shelf.

At least one of the side panels is openable to allow for the removal of a medical dressing from the package. As illustrated in figures 1 and 2, one of the side panels (denoted 103a) comprises a perforating line 112 that may be broken prior to removal of a dressing from the package.

As used herein, the term "set of printed product identifying elements" means at least three printed product identifying elements.

That the product identifying elements are "associated with the medical dressings" means that the printed elements convey product identifying or product descriptive characteristics or features indicative of the specific dressings stored in the package.

A "product identifying element" may be a textural or non-textural element that conveys information about the dressing contained in the package. The product identifying element may be a text element, e.g. a texted word, such as a brand name, a sub-brand, a product descriptor, a size indication etc. The product identifying element may also be a graphical element, e.g. a pictorial representation, a figure, image, photograph, sign, symbol or visual element that conveys information about the dressing and/or the use of the dressing in the package.

The product identifying elements are printed on the package; i.e. on the front panel and on the side panels, and optionally on the back panel. The printed product identifying elements may also be complemented with an embossed pattern, a weld pattern or a compressed pattern.

The product identifying elements may be printed onto the package by any conventional printing technique known in the art, including, but not limited to a gravure printing, a flexographic printing, an offset printing, an ink jet printing, laser printing and the like.

The first text element is typically a brand name. The first text element is generally the main brand name.

The dressing assortment connected to a main brand may be vast. One brand may contain hundreds of different dressings in sub-branded, size specific, and dressing category specific alternative versions. To identify the particular dressing of a main brand, the first text element and the first graphical element secure that the medical personnel can easily and quickly identify the product contained in the package and being suitable for a specific wound or specific care scenario.

To improve the recognition and product association, the first graphical element 104a-e may be an image, preferably a photograph, of the medical dressing.

Preferably, the first graphical element is a colored image or photograph of the medical dressing. In comparison, a colored image or photograph of the medical dressing, as used herein is different from a picture of a medical dressing per se. Usually pictures of medical dressings are fully computer rendered schematic and two-dimensional figures, usually in black and white, which at the most outlines a general shape of the medical dressing. However, a colored image or photograph, have the advantages of also providing additional information about the specific product (e.g. the product thickness), thereby facilitating the product selection process and product use clarification when used as disclosed herein.

A colored image or photograph allows the caregiver to easily and quickly recognize and identify the dressing contained in the package.

Preferably, the first graphical element is a color photograph of the medical dressing in the package, i.e. of an identical medical dressing disclosed in the package.

The term "photograph" as used herein is an image taken by a camera. It should be noted that a photograph can of course be digitally treated before printing it on the package.

At least one of the first graphical element visualizing the medical dressing is preferably a three dimensional image of the product. For example, the first graphical element disposed on the front panel may be a three dimensional image. If the medical dressing comprises a wound pad, the three dimensional image may enhance the impression of the thickness and/or degree of absorbency of the product.

The set of printed product identifying elements may also comprise at least a second graphical element 106a-e indicating a dressing property, preferably the absorption level associated with the medical dressings comprised in the package 100, and wherein the second graphical element 106a-e is disposed on the front panel 101 and on each one of the side panels 103a-d of the package.

In this context, the "absorption level" is typically a relative dressing property, indicating the level or degree of absorption ranging between a minimum absorption level and a maximum absorption level.

As illustrated in figure 1, the second graphical element 106a-e indicating the absorption level is illustrated as three drops, which may be filled, partially filled or non-filled depending on the absorption capacity of the dressing. As can be seen in figure 1, the three drops are filled, which indicates a maximum absorption level of the dressing.

The caregiver may thus quickly and clearly draw conclusions, when viewing the package on the shelf, the level of absorption associated with the dressings and thus easily select a dressing suitable for a specific wound to be treated.

The set of printed product identifying elements may further comprise at least a second text element 107a-e indicating the size of the medical dressings in the package 100, and wherein the second text element 107a-e is disposed on the front panel 101 and on each one of the side panels 103a-d of the package.

The size of the product is also an important feature for the medical personnel in their decision as to what product is to be selected for a specific patient, a specific wound, and a specific care scenario.

As illustrated in figure 1, the set of printed product identifying elements may also comprise additional printed product identifying elements, such as a sub-brand 113a-e, a texted product description 114 and/or a water tolerance indicator 115. The additional product identifying elements may also indicate the ability of the dressing to transfer exudate efficiently, remove bacterial biofilms, deal with pressure, shear, friction, microclimate etc and/or indicate that the product can be cut..

The set of printed product identifying elements may also comprise a graphical element, e.g. a pictorial representation, illustrating the size of the wound pad or size of the dressing (see 117 in figures 1 and 2).

To facilitate the selection of a medical dressing within a specific dressing category, the front panel 101 and/or the side panel(s) 103a-d may suitably comprise a color coded portion 108a-e; the color coded portion 108a-e being indicative of a specific medical dressing category.

The color coded portion 108a of the front panel is illustrated as white in figure 1 and 2 for ease of illustration. The same applies to the color coded portions 108b-e in figure 2.

The color coded portion 108a-e may indicate a particular genre or category of medical dressings. The color coded portion may be arranged in an edge portion of the of the front panel of the package. For example, the color coded portion may be arranged in an upper corner of the front panel 101 of the package 100 (denoted 108a in figure 2). The color coded portion draws the attention of the medical personnel and allows them to distinguish dressings belonging to different categories from one another.

The color coded portion 108a-e may be correlated with the first graphical element 105a-e or the second graphical element 106a-e.

A grey color coded portion may indicate that the dressings stored in the package are antimicrobial dressings.

For example, if the first graphical element visualizing the medical dressing is a color image or color photograph, the grey color of the wound pad of the dressing typically indicates that the dressing is an antimicrobial dressing. Hence, the color coded portion 108a-e may be grey to enhance the visual signal of this specific dressing category (antimicrobial dressings).

It is also conceivable that the color coded portion is associated with the second graphical element 106a-e, preferably indicating the absorption level of the dressing. In this case, the color of two or more dressing categories (typically not antimicrobial) may be the same, but the shade of the color may vary.

A high absorption dressing may be associated with a color coded portion with a darker color shade. A low absorption dressing may be associated with a color coded portion with a lighter color shade.

For example, the color coded portion may be light green if the absorption level is low, and green or dark green if the absorption level is high.

Preferably, each one of the side panels comprises a color coded portion 108be, the color coded portion being indicative of a specific medical dressing category.

By displaying the color coded portion 108b-e on the side panels 103a-d, the medical personnel is offered direct and quick guidance as to what type of dressing to select and use for a specific care scenario.

As illustrated in figures 1 and 2, the first graphical element 105a-d disposed on each one of the side panels 103a-d is arranged in the color coded portion 108b-e.

In exemplary embodiments, the side panels 103a-d have a background color that contrasts with the background color of the front panel 101, and, optionally, of the back panel 102.

The front panel typically has a lighter background color, e.g. white. This is beneficial since it allows for the plurality of graphical elements, text elements and product identifying means on the front panel to be visualized and easily recognized.

The side panels 103a-d may have a background color that contrasts with the background color of the front panel 101, and, optionally, of the back panel 102.

The background color of the side panels 103a-d typically corresponds to the color of the first text element 104a-e; i.e. the brand name disposed on the front panel 101. This yields a cohesive impression and qualitative appearance, and also facilitates the identification of the packages on a shelf.

The background color of the side panels 103a-d is preferably green, and the background color of the front panel 101 is preferably white.

In preferred embodiments, the side panels 103a-d are visually correlated.

As used herein the term "visually correlated" means that at least 80% of the printed product identifying elements disposed on a side panel correspond to the printed product identifying elements disposed on the other side panels. Typically, the background color of each side panel is the same.

The front panel 101 may comprise a larger number of printed product identifying elements than the side panels 103a-d.

A more comprehensive set of product identifying and descriptive texted and graphical elements are desirably present on the front panel, which is the panel viewed by the medical personnel upon dressing removal.

Each one of the side panels may contain four printed product identifying elements associated with the medical dressings, and wherein the product identifying elements are disposed on the side panels in a visually correlated arrangement.

In this context, "visually correlated arrangement", means that at least 80% of the printed product identifying elements disposed on a side panel correspond to the printed product identifying elements disposed on the other side panels. At least 80% of the printed product identifying elements are also disposed on each side panel in substantially similar positions.

As best illustrated in figure 2, each side panel contains the same product identifying elements.

Typically, the background color of each side panel is the same.

Typically, each side panel comprises three to six printed product identifying elements, preferably four to five printed product identifying elements.

If too many product identifying elements are disposed on the side panels, the impression may be "disturbed" and it may be difficult to identify the key product identifying elements of the medical dressing stored in a specific package.

The set of printed product identifying elements may also comprise a tag 109 being readable using an electronic device.

Preferably, the tag is optically readable using an electronic device.

The tag holds identifiable information that is readable, using an electronic device. The tag may be read/scanned by the electronic device, e.g. a mobile electronic device. Typically, the tag is a one-dimensional (ID) or a two-dimensional (2D) bar code. In general, another technology for reading a tag could be through RFID technology, hence a tag used herein could e.g. be an RFID tag.

The optically readable tag or bar code may be captured by a camera comprised in the electronic device.

In scanning the tag; i.e. bar code, the medical personnel may gain access to more information about the specific dressing and its use in the treatment of a specific wound.

As illustrated in figure 1, the first graphical element 105a disposed on the front panel 101 visualizes at least a portion of a top layer 110 and at least a portion of a bottom layer 111 of the medical dressing.

Accordingly, the intended use of the product is communicated in a clear and simple manner to the medical personnel.

As illustrated in figure 1, the medical dressing is partially folded such that the personnel can view the dressing both from the top layer 110 and the bottom layer 111. In this view, at least a portion of the wound pad 116 is also illustrated.

It should be noted that the medical dressing may comprise a wound pad arranged between a top layer and a bottom layer. It is preferred that the first graphical element disposed on the front panel is visualizing a portion of the top layer, a portion of the wound pad, and a portion of the bottom layer of the medical dressing. This enables a user to readily understand the different features of the medical dressing in an easy manner. In this way at least three different materials are visualized in the first graphical element disposed on the front panel. The wound pad is preferably visible through the bottom layer of the medical dressing.

It is preferred that the first graphical element visualizes the top layer to a larger extent than the bottom layer and the wound pad as this is generally the layer or side that a user, e.g. a nurse, identifies the specific wound dressing by.

As used herein in, the top layer is usually a breathable liquid impermeable layer. The bottom layer is typically an adhesive layer adapted to be positioned against, and adhere to, a user's skin during use.

Each one of the medical dressings comprised in the package is typically individually stored in a sterile pouch.

The sterile pouch keeps the dressing in a sterile condition prior to use.

The sterile pouch may comprise a second set of printed product identifying elements associated with the medical dressing, and wherein at least two product identifying elements of the second set of printed product identifying elements correspond to at least two product identifying elements of the set of printed product identifying elements of the package.

The second set of printed product identifying elements may also contain additional product identifying information. For example, the second set of printed product identifying elements may comprise at least one pictorial representation of a step associated with the correct application of the medical dressing onto the body part.

The second set of printed product identifying elements may comprise a pictorial representation, i.e. an image or figure, that illustrates a step associated with the application of the medical dressing to the skin in clear and simple manner.

The present disclosure also relates to a flat box for packaging of medical dressings. The flat box is best illustrated in figure 2. This represents the configuration that the package has prior to folding it into a package as disclosed in figure 1.

The flat box comprises a front panel 101, a back panel 102, and four side panels 103a-d, wherein the four side panels 103a-d are arranged around the front panel 101 or the back panel 102; the front panel 101 being connected to the back panel 102 by one of the four side panels 103a-d, wherein the flat box comprises a set of printed product identifying elements 104a-e, 105a-e, 106a-e, 107a-e associated with the medical dressings to be packaged in the flat box, wherein the set of printed product identifying elements comprises at least a first text element 104a-e, preferably a brand name, and a first graphical element 105a-e visualizing a medical dressing to be packaged in the flat box 100, wherein the first text element 104-e and the first graphical element 105a-e are disposed on the front panel 101 and on each one of the side panels 103a-d of the flat box.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A package (100) comprising a plurality of medical dressings, wherein said package (100) comprises a front panel (101), an opposing back panel (102) and four side panels (103a-d) extending between said front panel (101) and said back panel (102), wherein said package comprises a set of printed product identifying elements (104a-e, 105a-e, 106a-e, 107a-e) associated with the medical dressings in said package, wherein said set of printed product identifying elements comprises at least a first text element (104a-e), preferably a brand name, and a first graphical element (105a-e) visualizing a medical dressing in said package (100), wherein said first text element (104-e) and said first graphical element (105a-e) are disposed on said front panel (101) and on each one of said side panels (103a-d) of said package (100).

2. The package (100) according to claim 1, wherein said first graphical element (105a-e) is an image, preferably a photograph, of said medical dressing.

3. The package (100) according to claim 1 or claim 2, wherein said set of printed product identifying elements comprises at least a second graphical element (106a-e) indicating a dressing property, preferably the absorption level, associated with the medical dressings comprised in said package (100), and wherein said second graphical element (106a-e) is disposed on said front panel (101) and on each one of said side panels (103a-d) of said package (100).

4. The package (100) according to any one of the preceding claims, wherein said set of printed product identifying elements comprises at least a second text element (107a-e) indicating the size of the medical dressings in said package (100), and wherein said second text element (107a-e) is disposed on said front panel (101) and on each one of said side panels (103a-d) of said package (100).

5. The package (100) according to any one of the preceding claims, wherein said front panel (101) and/or said side panel(s) (103a-d) comprises at least one color coded portion (108a-e); said color coded portion (108a-e) being indicative of a specific medical dressing category.

6. The package (100) according to claim 5, wherein said color coded portion (108a-e) is correlated with said first graphical element (105a-e) or said second graphical element (106a-e).

7. The package according to any one of the preceding claims, wherein each one of said side panels (103a-d) comprises a color coded portion (108b-e), said color coded portion (108b-e) being indicative of a specific medical dressing category.

8. The package according to claim 7, wherein said first graphical element (105b-e) disposed on each one of said side panels (103a-d) is arranged in said color coded portion (108b-e).

9. The package (100) according to any one of the preceding claims, wherein said side panels (103a-d) have a background color that contrasts with the background color of said front panel (101), and, optionally, of said back panel (102).

10. The package (100) according to any one of the preceding claims, wherein said side panels (103a-d) are visually correlated.

11. The package (100) according to any one of the preceding claims, wherein said front panel (101) comprises a larger number of printed product identifying elements than said side panels (103a-d).

12. The package (100) according to any one of the preceding claims, wherein each one of said side panels (103a-d) contains four printed product identifying elements (104a-e,105a-e,106a-e,107a-e) associated with the medical dressings in said package, and wherein said product identifying elements are disposed on said side panels (103a-d) in a visually correlated arrangement.

13. The package (100) according to any one of the preceding claims, wherein said set of printed product identifying elements comprises a tag (109) being readable using an electronic device, wherein said tag (109) is disposed on said front panel (101) of said package.

14. The package (100) according to any one of the preceding claims, wherein said first graphical element (105a) disposed on said front panel (101) visualizes at least a portion of a top layer (110) and at least a portion of a bottom layer (111) of said medical dressing.

15. The package (100) according to any one of the preceding claims, wherein each one of the medical dressings comprised in said package is individually packaged in a sterile pouch.

16. The package (100) according to claim 15, wherein said sterile pouch comprises a second set of printed product identifying elements associated with said medical dressing, and wherein at least two product identifying elements of said second set of printed product identifying elements correspond to at least two product identifying elements of said set of printed product identifying elements of said package (100).

17. A flat box for packaging of medical dressings, said flat box comprising a front panel (101), a back panel (102), and four side panels (103a-d), wherein said four side panels (103a-d) are arranged around said front panel (101) or said back panel (102); said front panel (101) being connected to said back panel (102) by one of said four side panels (103a-d), wherein said flat box comprises a set of printed product identifying elements (104a-e, 105a-e, 106a-e, 107a-e) associated with the medical dressings to be packaged in said flat box, wherein said set of printed product identifying elements comprises at least a first text element (104a-e), preferably a brand name, and a first graphical element (105a-e) visualizing a medical dressing to be packaged in said flat box (100), wherein said first text element (104-e) and said first graphical element (105a-e) are disposed on said front panel (101) and on each one of said side panels (103a-d) of said flat box.
